Europäisches Patentamt

European Patent Office

Office européen des brevets

(19)

(11) Veröffentlichungsnummer: **0 084 769**
**B1**

# (12) EUROPÄISCHE PATENTSCHRIFT

(45) Veröffentlichungstag der Patentschrift:
30.04.86

(21) Anmeldenummer: 83100020.3

(22) Anmeldetag: 04.01.83

(51) Int. Cl.⁴: **C 08 F 2/44**, C 08 F 220/20

(54) Vernetzte, füllstoffhaltige Perlpolymerisate und Verfahren zu ihrer Herstellung.

(30) Priorität: 15.01.82 DE 3201109

(43) Veröffentlichungstag der Anmeldung:
03.08.83 Patentblatt 83/31

(45) Bekanntmachung des Hinweises auf die Patenterteilung:
30.04.86 Patentblatt 86/18

(84) Benannte Vertragsstaaten:
AT BE CH DE FR GB IT LI LU NL SE

(56) Entgegenhaltungen:
EP - A - 0 011 190
GB - A - 1 456 865

Die Akte enthält technische Angaben, die nach dem Eingang der Anmeldung eingereicht wurden und die nicht in dieser Patentschrift enthalten sind.

(73) Patentinhaber: BAYER AG, Konzernverwaltung RP
Patentabteilung, D-5090 Leverkusen 1 Bayerwerk (DE)

(72) Erfinder: Podszun, Wolfgang, Dr., Wolfskaul 4,
D-5000 Köln 80 (DE)
Erfinder: Walkowiak, Michael, Dr.,
Albertus-Magnus-Strasse 10, D-5090 Leverkusen 1 (DE)
Erfinder: Süling, Carlhans, Dr.,
Carl-Leverkus-Strasse 10, D-5068 Odenthal (DE)

## Beschreibung

Gegenstand der Erfindung sind vernetzte, transparente, gleichmäßig mit anorganischem Füllstoff auf Siliziumdioxidbasis gefüllte Perlpolymerisate mit einer mittleren Teilchengröße von 5 bis 150 µm.

Die Herstellung von ungefüllten Perlpolymerisaten ist zum Beispiel aus Houben-Weyl, Methoden der Organischen Chemie, Band XIV/1, bekannt. Man dispergiert dazu olefinisch ungesättigte Monomere in Wasser unter Rühren und polymerisiert die gebildeten Tröpfchen mit Hilfe eines monomerlöslichen Initiators.

Um ein Koagulieren des Reaktionsansatzes während der Polymerisation zu verhindern, müssen im allgemeinen Dispergatoren eingesetzt werden; dies sind in der Regel wasserlösliche, hochmolekulare, organische Verbindungen oder feinteilige, wasserunlösliche Verbindungen (zu letzteren gehören z.B. die als Pickering-Dispergatoren bezeichneten Substanzen).

Die Polymerisation von feinteilige, unlösliche, anorganische Füllstoffe enthaltenden Monomeren führt in der Regel nicht zu Perlen, die diese Partikel in über den Querschnitt gleichmäßig verteilte Form enthalten, da die anorganischen Partikel als Dispergator wirken und an die Wasser-Monomer Phasengrenze wandern.

In der US-A- 3 716 505 wird die Suspensionspolymerisation von Mischungen aus Vinylmonomeren, einem im Monomeren löslichen Polymerisat und einer unlöslichen anorganischen Verbindung, wie zum Beispiel Siliziumdioxid, beschrieben. Auch in diesem Falle wirkt die anorganische Verbindung als Dispergator und befindet sich nach der Polymerisation auschließlich an der Perlenoberfläche.

Die DE-A- 28 49 936 beschreibt die Herstellung von Perlen, die feinteilige Füllstoffe in gleichmäßig verteilter Form enthalten. Dieses Verfahren ist durch die Verwendung von Monomeren oder Monomer-Polymerisat-Mischungen mit einer erhöhten Viskosität (0,1 bis 10 Pa.s, gemessen bei der Dispergiertemperatur) gekennzeichnet. Nach dieser Verfahrenseweise lassen sich auch vernetzte SiO$_2$-haltige Perlen erzeugen, jedoch weisen diese Produkte häufig eine geringe Transparenz auf, was bei speziellen Anwendungen von Nachteil sein kann.

Es wurde nunmehr überraschenderweise gefunden, daß man das Problem der mangelnden Transparenz solcher Siliziumdioxid enthaltenden Perlen in einfacher Weise lösen kann, indem man silanisierte Füllstoffe einsetzt.

Gegenstand der Erfindung sind vernetzte, transparente, gleichmäßig mit einem anorganischen Füllstoff gefüllte Perlpolymerisate mit einer mittleren Teilchengröße von 5 bis 150 µm, welche erhältlich sind, indem man eine Mischung (I), enthaltend

(A) 10-60 Gew.-%, vorzugsweise 15-45 Gew.-% (bezogen auf gesamte Mischung) eines Füllstoffes auf Siliziumdioxidbasis,

(B) 2-90 Gew.-%, vorzugsweise 2-25 Gew.-% eines Di(meth)acrylats und/oder eines Tri(meth)acrylats,

(C) 0-88 Gew.-%, vorzugsweise 30-83 Gew.-% eines Mono(meth)acrylats und

(D) 0-20 Gew.% an weiteren Monomeren, mit einem Polymerisationsinitiator aktiviert, in einer wäßrigen Lösung (II) eines hochmolekularen organischen Dispergators dispergiert und die Dispersion durch Erhitzen über die Zerfallstemperatur des Initiators polymerisiert. Die erfindungsgemäßen Perlpolymerisate sind dadurch gekennzeichnet, daß der Füllstoff (A) eine mittlere Teilchengröße von 5 bis 100 nm aufweist und mit 1 bis 25 Gew.-% einer Silanverbindung behandelt ist.

Als Füllstoff (A) ist erfindungsgemäß Siliziumdioxid besonders bevorzugt. Das Siliziumdioxid kann beispielsweise durch Fällung oder durch Flammhydrolyseverfahren hergestellt werden. Neben reinem Siliziumdioxid können auch Mischoxide des Siliziumdioxids, beispielsweise mit Aluminiumoxid, Boroxid, Titandioxid oder Zirkonoxid, eingesetzt werden, sofern der SiO$_2$-Anteil im Mischoxid höher als 50 Gew.-%, vorzugsweise höher als 75 Gew.-%, ist. Besonders gut geeignet ist flammhydrolytisch gewonnenes Siliziumdioxid mit einer mittlere Teilchen-größe (Primärteilchengröße) von 10 bis 40 nm und einer BET-Oberfläche von 30 bis 300, vorzugsweise 40 bis 200 m$^2$/g.

Der Füllstoff auf Siliziumdioxidbasis wird vor seiner Verwendung zur Herstellung der erfindungsgemäßen Perlen oberflächenbehandelt. Geeignete Oberflächenbehandlungsmittel sind in erster Linie die als Haftvermittler an sich bekannten Silanverbindungen, die beispielsweise in den US-PSen 30 66 113 und 35 39 533 beschrieben werden. Es können sowohl gesättigte Silanverbindungen, wie beispielsweise Hexamethyldisilazan oder γ-Glycidoxypropyltrimethoxysilan, als auch ungesättigte Silanverbindungen angewendet werden.

Als ungesättigte, polymerisierbare Silanverbindungen seien beispielhaft genannt: Vinyltriethoxysilan, Vinyltrimethoxysilan, γ-Methacryloxypropyltrimethoxysilan, γ-Methacryloxypropyl-tris(2-methoxyethoxy)-silan und Vinyltriacetoxysilan.

Die Silanverbindung soll in Anteilen von 1 bis 25, vorzugsweise von 5 bis 20 Gew.-%, bezogen auf den Füllstoff auf Siliziumdioxidbasis, angewendet werden. Die Silanisierungsreaktion wird im allgemeinen in einem inerten Lösungsmittel, beispielsweise in Methylenchlorid oder Toluol, durchgeführt, doch ist es in manchen Fällen, zum Beispiel bei der Nachbehandlung mit Hexamethyldisilazan, auch möglich, auf ein Lösungsmittel zu verzichten.

Für die Herstellung der erfindungsgemäßen Perlpolymerisate kommen beliebige an sich bekannte olefinisch ungesättigte Monomere der oben genannten Art in Betracht.

Als Di(meth)acrylate (B) seien beispielhaft erwähnt: Ethylendi(meth)acrylat,

Diethylenglykoldi(meth)acrylat, Triethylenglykoldi(meth)acrylat, Neopentylglykoldi(meth)acrylat, 1,12-Dodekandi(meth)acrylat, ferner Derivate des Bisphenol-A, wie 2,2-Bis-[4(2-hydroxy-3-methacryloyloxypropyl)-phenyl]-propan (Bis-GMA), sowie Urethandi(meth)acrylate, wie sie zum Beispiel in den US-PSen 34 25 988, 37 09 866 und 36 29 187 beschrieben sind.

Als Tri(meth)acrylate kommen zum Beispiel in Frage: Glycerintri(meth)acrylat, Trimethylolpropantri(meth)acrylat und Pentaerythrittri(meth)acrylat.

Als Mono-(meth)acrylate (C) können beispeilsweise verwendet werden: Methyl(meth)acrylat, Ethyl(meth)acrylat, Butyl(meth)acrylat, Lauryl(meth)acrylat, Cyclohexyl(meth)acrylat und Dihydrodicyclopentadienyl-(meth)acrylat.

Mit der Bezeichnung (Meth)acrylat sind sowohl das Methacrylat als auch das Acrylat gemeint. Zur Herstellung der erfindungsgemäßen Perlpolymerisate werden vorzugsweise die Methacrylate eingesetzt.

Neben den (Meth)acrylaten können bis zu 20 Gew.-% (bezogen auf die Summe aus Füllstoff und den Monomeren) weitere Vinyl- bzw. Vinyliden-Monomere, wie beispielsweise Styrol, α-Methylstyrol, Acrylnitril oder Vinylacetat eingesetzt werden.

Zur Herstellung der erfindungsgemäßen Perlpolymerisate ist es nicht notwendig, besonders hochviskose Monomere einzusetzen. Die Viskosität der verwendeten Monomeren, das heißt die Viskosität der Mischung aus (B), (C) und (D), liegt vorzugsweise im Bereich von 0,001 bis 0,09 Pa.s (1 - 90 Centipoise) bei der Dispergiertemperatur.

Die Mischung aus Füllstoff und den genannten Monomeren kann in üblichen Rührapparaturen hergestellt werden. Es sollen dabei vorzugsweise hohe Scherkräfte, zum Beispiel Rührenergien von 1 bis 10 Watt/l, angewendet werden.

Während des Mischvorganges oder nach Abschluß des Mischens wird vorzugsweise unter Rühren ein Vakuum von (0,013 bis 400 mbar), besonders bevorzugt von (1,33 bis 133 mbar), angelegt. Die Vakuumbehandlung, die mindestens einige Minuten, beispielsweise mindestens 10 Minuten, stattfinden soll, erfolgt vorzugsweise bei Raumtemperatur, doch können auch höhere oder niedrigere Temperaturen angewendet werden. Es ist dabei von Vorteil, wenn beim Evakuieren ein geringer Bruchteil der eingesetzten Monomeren (0,01 bis 5 %) abdestilliert wird, da auf diese Weise Wasserspuren aus den Monomeren und von der Siliziumdioxidoberfläche entfernt werden können. Zweckmäßigerweise wird die Füllstoff-Monomermischung mit Inertgas, zum Beispiel Stickstoff, belüftet.

Zur Aktivierung können übliche monomerlösliche Radikalbildner verwendet werden; beispielhaft seien genannt: Peroxidund Azoverbindungen, wie Dibenzoylperoxid, Dilauroylperoxid, Cyclohexylpercarbonat und Azoisobuttersäuredinitril. Gut geeignet sind auch Mischungen von Polymerisationsinitiatoren mit unterschiedlichen Zerfallstemperaturen. Die Zugabe des Polymerisationsinitiators kann vor oder nach dem Evakuierungsschritt erfolgen. Um vorzeitiges Anpolymerisieren zu vermeiden, ist es jedoch zweck-mäßig, die Starter erst umittelbar vor dem Dispergieren zuzusetzen.

Das aktivierte Siliziumdioxid-Monomer-Gemisch wird mittels einer wäßrigen Polymerisatlösung dispergiert. Um möglichst gleichmäßige und transparente Perlen zu erzeugen, ist es vorteilhaft, die Dispergatorlösung vorzulegen und das Füllstoff-Monomergemisch langsam unter Rühren zuzusetzen.

Das Verhältnis von Monomerphase zu Wasserphase beträgt in der Regel 1:1 bis 1:10, vorzugsweise 1:2 bis 1:5.

Als Dispergatoren sind alle zu diesem Zweck an sich bekannten wasserlöslichen makromolekularen Verbindungen, z.B. Cellulosederivate, wie Methylcellulose, und teilverseifte Polyvinylacetate geeignet. Gut geeignet sind auch Copolymerisate aus (Meth)acrylsäure und (Meth)acrylsäurealkylester. Besonders bevorzugt wird die alkalische Lösung eines Copolymerisates aus Methacrylsäure und Methylmethacrylat. Der Gehalt an Dispergator soll vorzugsweise 0,5 bis 5 Gew.-%, bezogen auf die Wasserphase, betragen.

Die Polymerisation wird durch Erhitzen auf die Zerfallstemperatur des Polymerisationsinitiators eingeleitet. Die Reaktion soll so geführt werden, daß die Monomeren nicht zum Sieden kommen. Bei einsetzender exothermer Reaktion muß gegebenenfalls gekühlt werden. Es ist vorteilhaft, die Polymerisation unter erhöhtem Druck, beispielsweise unter 2 bis 6 bar Stickstoffdruck, durchzuführen.

Aus der auspolymerisierten Dispersion kann auf bekannte Weise durch Dekantieren, Filtrieren, Waschen und Trocknen das Perlpolymerisat gewonnen werden.

Die erfindungsgemäßen Perlpolymerisate eignen sich in hervorragender Weise als Komponenten von Dentalmaterialien, insbesondere von Zahnfüllungsmaterialien. Aus Perlpolymerisaten lassen sich durch Abmischen mit monomeren Bindern pastöse Zubereitungen erhalten, die gute Verarbeitungseigenschaften aufweisen und durch Härtung in Werkstoffe von hoher Transparenz und hoher mechanischer Festigkeit übergeführt werden können.

**Beispiel 1**

Silanisierung von Siliziumdioxid
In einem Rührkessel werden
4 l Aceton
37,5 g γ -Methacryloxypropyltrimethoxysilan
0,5 g Dicyclohexylamin
10 g destilliertes Wasser
vorgelegt. Unter Rühren werden 462,5 g Siliziumdioxid (mittlere Teilchengröße 30 nm, BET-Oberfläche 120 m²/g) zugegeben und 2 Stunden unter Rückfluß gerührt. An-schließend wird das Aceton abdestilliert. Der Rückstand wird 15 Stunden bei 60°C und anschließend weitere 6 Stunden bei 90°C getrocknet. Kohlenstoffgehalt des Produktes: 2,2 % (naß C).

**Beispiel 2**

Silanisierung von Siliziumdioxid

In einem Rührkessel werden 1500 g Siliziumdioxid (Aerosil ® OX 50 der Firma Degussa) gegeben und unter starkem Rühren 265 g Hexamethyldisilazan langsam zugetropft. Anschließend wird so lange unter schwachem Vakuum gerührt, bis kein Ammoniak nachweisbar ist.

Kohlenstoffgehalt: 0,95 % (naß C)

**Beispiel 3**

Herstellung eines vernetzten, Siliziumdioxidhaltigen Perlpolymerisates

a) Siliziumdioxid-Monomer-Mischung

In einem 40-Liter-Rührkessel werden

3600 g silanisiertes Siliziumdioxid aus Beispiel 1

9360 g Methylmethacrylat

4320 g Isobutylmethacrylat

720 g Bis-GMA

15 Minuten lang unter intensivem Rühren gemischt und unter weiterem Rühren 5 Minuten lang einem Vakuum von ca. 20 mbar ausgesetzt; anschließend wird mit Stickstoff belüftet.

Die Mischung wird unmittelbar vor der Weiterverarbeitung mit 72 g Benzoylperoxid und 72 g Cyclohexylpercarbonat versetzt und durch Erhöhen des $N_2$-Druckes über eine Steigleitung in den vorbereiteten 120-Liter-Reaktions-autoklaven gedrückt.

b) Polymerisation

In einem 120-Liter-Autoklaven mit Ankerimpeller-Rührer werden unter Stickstoffspülung 50,4 kg destilliertes Wasser und 21,6 kg Dispergatorlösung (8,5 % wäßrige, NaOHalkalische Lösung eines Copolymerisates aus gleichen Gewichtsteilen Methylmethacrylat und Methacrylsäure) vorgelegt und 5 Minuten gemischt. Bei laufendem Rührer (250 UmM) wird das oben beschriebene Monomergemisch innerhalb von 15 Minuten eingedrückt. Nach beendeter Zugabe wird 2 Stunden bei Raumtemperatur gerührt. An-schließend wird 5 bar $N_2$ aufgedrückt und die Temperatur innerhalb von 15 Minuten auf 75°C (innen) erhöht. Bei einsetzender exothermer Reaktion (ca. 30 Minuten nach Aufheizbeginn) wird so stark gekühlt, daß die Innentemperatur im Bereich von 78 bis 85°C gehalten wird. Nach dem Abklingen der Reaktion wird 3 Stunden bei 80°C nachgerührt. Das feste Perlpolymerisat wird nach dem Abkühlen durch Abdekantieren von feinteiligen Anteilen befreit, anschließend filtriert, mehrfach mit destilliertem Wasser gewaschen und bei 80°C getrocknet. Man erhält 15 000 g vollständig transparente Perlen mit einem mittleren Teilchendurchmesser von 25 µm und einem $SiO_2$-Gehalt von 20,5 %.

**Beispiele 4 und 5**

Beispiel 3 wird unter Verwendung einer Siliziumdioxid-Monomer-Mischung aus

7200 g silanisiertem Siliziumdioxid aus Beispiel 2

9720 g Methylmethacrylat

4320 g Ethylacrylat und

360 g Ethylendimethacrylat (Beispiel 4) bzw. aus

5400 g Siliziumdioxid (mittlere Teilchengröße 30 nm, BET-Oberfläche 170 m²/g, silanisiert mit 5 Gew.-% Hexamethyldisilazan),

10080 g Methylmethacrylat

2160 g n-Butylmethacrylat

2160 g Bis-GMA (Beispiel 5)

wiederholt. Es werden jeweils ca. 15 kg transparente Perlen erhalten

|  | (4) | (5) |
|---|---|---|
| mittlerer Teilchendurchmesser | 35µm | 48µm |
| $SiO_2$-Gehalt | 34 % | 27,5 % |

**Patentansprüche**

1) Vernetzte, transparente, gleichmäßig mit einem anorganischen Füllstoff gefüllte Perlpolymerisate mit einer mittleren Teilchengröße von 5 bis 150 µm, welche erhältlich sind, indem man eine Mischung (I), enthaltend

(A) 10 - 60 Gew.-% eines Füllstoffes auf Siliziumdioxidbasis,

(B) 2 - 90 Gew.-% eines Di-(meth)acrylats und/oder eines Tri(meth)acrylats,

(C) 0 - 88 Gew.-% eines Mono(meth)acrylats und

(D) 0 - 20 Gew.-% an weiteren Monomeren,

mit einem Polymerisationsinitiator aktiviert, in einer wäßrigen Lösung (II) eines hochmolekularen organischen Dispergators dispergiert und die Dispersion durch Erhitzen auf die Zerfallstemperatur des Initiators polymerisiert, dadurch gekennzeichnet, daß der Füllstoff (A) eine mittlere Teilchengröße von 5 bis 100 nm aufweist und mit 1 bis 25 Gew.-% einer Silanverbindung behandelt ist.

2) Perlpolymerisate nach Anspruch 1, dadurch gekennzeichnet, daß vor ihrer Herstellung die Mischung (I) einer Vakuumbehandlung unterzogen wird.

3) Perlpolymerisate nach Anspruch 1 oder 2, dadurch gekennzeichnet, daß zu ihrer Herstellung die Mischung (I) in der Lösung (II) dispergiert wird, indem man Lösung (II) vorlegt und Mischung (I) langsam unter Rühren zufügt.

4) Perlpolymerisate nach Anspruch 1 bis 3, dadurch gekennzeichnet, daß die Viskosität der Mischung aus (B), (C) und (D) 0,001 bis 0,09 Pa.s beträgt.

5) Perlpolymerisate nach Anspruch 1 bis 4, dadurch gekennzeichnet, daß Lösung (II) eine alkalische Lösung eines Methacrylsäure/Methacrylsäuremethylester-Copolymerisates ist.

6) Verfahren zur Herstellung von vernetzten, transparenten, gleichmäßig mit einem anorganischen Füllstoff vernetzten Perlpolymerisaten mit einer mittleren Teilchengröße von 5 bis 150 µm, indem man eine Mischung (I), enthaltend

(A) 10 - 60 Gew.-% eines Füllstoffes auf Siliziumdioxidbasis,

(B) 2 - 90 Gew.- % eines Di-(meth)acrylats und/oder eines Tri(meth)acrylats,

(C) 0 - 88 Gew.-% eines Mono(meth)acrylats und

(D) 0 - 20 Gew.% an weiteren Monomeren,

mit einem Polymerisationsinitiator aktiviert, in einer wäßrigen Lösung (II) eines hochmolekularen organischen Dispergators dispergiert und die Dispersion durch Erhitzen auf die Zerfallstemperatur des Initiators polymerisiert, dadurch gekennzeichnet, daß der Füllstoff (A) eine mittlere Teilchengröße von 5 bis 100 nm aufweist und mit 1 bis 25 Gew.-% einer Silanverbindung behandelt ist.

7) Verfahren nach Anspruch 6, dadurch gekennzeichnet, daß die Mischung (I) vor der Dispergierung einer Vakuumbehandlung unterzogen wird.

8) Verfahren nach Anspruch 6 oder 7, dadurch gekennzeichnet, daß die Mischung (I) in der Lösung (II) dispergiert wird, indem man Lösung (II) vorlegt und Mischung (I) langsam unter Rühren zufügt.

9) Verfahren nach Anspruch 6 bis 8, dadurch gekennzeichnet, daß die Viskosität der Mischung aus (B) (C) und (D) 0.001 bis 0.09 Pa.s beträgt.

10) Verfahren nach Anspruch 6 bis 9, dadurch gekennzeichnet, daß Lösung (II) eine alkalische Lösung eines Methacrylsäure/Methacrylsäuremethylester-Copolymerisates ist.

## Claims

1. Crosslinked, transparent bead polymers having an average particle size of 5 to 150 μm and uniformly filled with an inorganic filler, which are obtained by activating a mixture (I) containing

(A) 10 - 60% by weight of a filler based on silicon dioxide,

(B) 2 - 90% by weight of a di(meth)acrylate and/or of a tri(meth)acrylate,

(C) 0 - 88% by weight of a mono(meth)acrylate and

(D) 0 - 20% by weight of other monomers,

with a polymerisation initiator, dispersing in an aqueous solution (II) of a high molecular weight organic dispersing agent and polymerising the dispersion by heating to the decomposition temperature of the initiator, characterised in that filler (A) has an average particle size of 5 to 100 nm and has been treated with 1 to 25% by weight of a silane compound.

2. Bead polymers according to Claim 1, characterised in that prior to the preparation thereof mixture (I) is subjected to a vacuum treatment.

3. Bead polymers according to Claim 1 or 2, characterised in that for the preparation thereof mixture (I) is dispersed in solution (II) by initially introducing solution (II) and slowly adding mixture (I) with stirring.

4. Bead polymers according to Claim 1 to 3, characterised in that the viscosity of the mixture of (B), (C) and (D) is 0.001 to 0.09 Pa.s.

5. Bead polymers according to Claim 1 to 4, characterised in that solution (II) is an alkaline solution of a methacrylic acid/ methyl methacrylate copolymer.

6. Process for the preparation of crosslinked, transparent bead polymers having an average particle size of 5 to 150 μm and uniformly crosslinked with aninorganicfiller,by activating a mixture (I) containing

(A) 10 - 60% by weight of a filler based on silicon dioxide,

(B) 2 - 90% by weight of a di(meth)acrylate and/or of a tri(meth)acrylate,

(C) 0 - 88% by weight of a mono(meth)acrylate and

(D) 0 - 20% by weight of other monomers,

with a polymerisation initiator, dispersing in an aqueous solution (II) of a high molecular weight organic dispersing agent and polymerising the dispersion by heating to the decomposition temperature of the initiator, characterised in that the filler (A) has an average particle size of 5 to 100 nm and has been treated with 1 to 25% by weight of a silane compound.

7. Process according to Claim 6, characterised in that the mixture (I) is subjected to a vacuum treatment before the dispersion.

8. Process according to Claim 6 or 7, characterised in that mixture (I) is dispersed in solution (II) by initially introducing solution (II) and slowly adding mixture (I) with stirring.

9. Process according to Claim 6 to 8, characterised in that the viscosity of the mixture of (B) (C) and (D) is 0.001 to 0.09 Pa.s.

10. Process according to Claim 6 to 9, characterised in that solution (II) is an alkaline solution of a methacrylic acid/ methyl methacrylate copolymer.

## Revendications

1. Produits de polymérisation en suspension réticulés, transparents, uniformément additionnés d'une charge inorganique, ayant une grosseur moyenne de particules de 5 à 750 μm, qui sont obtenus en activant un mélange (I), contenant

(A) 70 à 60 % en poids d'une charge à base de dioxyde de silicium,

(B) 2 à 90 % en poids d'un di-(méth)acrylate et/ou d'un tri(méth)acrylate,

(C) 0 à 88 % en poids d'un mono(méth)acrylate et

(D) 0 à 20 % en poids d'autres monoméres, avec un initiateur de polymérisation, en le dispersant dans une solution aqueuse (II) d'un agent dispersant organique de haut poids moléculaire et en polymérisant la dispersion par chauffage à la température de décomposition de l'initiateur, caractérisés en ce que la charge (A) présente une grosseur moyenne de particules de 5 à 700 nm et est traitée avec 7 à 25 % en poids d'un composé du type silane.

2. Produits de polymérisation en suspension suivant la revendication 1, caractérisés en ce que le mélange (I) est soumis à un traitement sous vide avant leur production.

3. Produits de polymérisation en suspension suivant la revendication 7 ou 2, caractérisés en ce que le mélange (I) est dispersé dans la solution (II) en vue de leur production, et on introduit alors préalablement la solution (II), puis on y ajoute lentement sous agitation le mélange (I).

4. Produits de polymérisation en suspension suivant les revendications 7 à 3, caractérisés en ce que la viscosité du mélange de (B), (C) et (D) s'élève à 0,001-0,09 Pa.s.

5. Produits de polymérisation en suspension suivant les revendications 7 à 4, caractérisés en ce que la solution (II) est une solution alcaline d'un copolymère acide méthacrylique/ester méthylique d'acide méthacrylique.

6. Procédé pour obtenir des produits de polymérisation en suspension réticulés, transparents, uniformément réticulés avec une charge inorganique, présentant un diamètre moyen des particules de 5 à 750 µm, par activation d'un mélange (I), contenant

(A) 70 à 60 % en poids d'une charge à base de dioxyde de silicium,

(B) 2 à 90 % en poids d'un di-(méth)acrylate et/ou d'un tri-(méth)acrylate,

(C) 0 à 88 % en poids d'un mono(méth)acrylate et

(D) 0 à 20 % en poids d'autres monomères, avec un initiateur de polymérisation, dispersion dans une solution aqueuse (II) d'un agent dispersant organique de haut poids moléculaire et polymérisation de la dispersion par chauffage à la température de décomposition de l'initiateur, caractérisé en ce que la charge (A) présente un diamètre moyen de particules de 5 à 700 nm et est traitée avec 7 à 25 % en poids d'un composé du type silane.

7. Procédé suivant la revendication 6, caractérisé en ce que le mélange (I) est soumis à un traitement sous vide avant la dispersion.

8. Procédé suivant la revendication 6 ou 7, caractérisé en ce que le mélange (I) est dispersé dans la solution (II) de telle sorte que la solution (II) est introduite au préalable et le mélange (I) est lentement ajouté sous agitation.

9. Procédé suivant les revendications 6 à 8, caractérisé en ce que la viscosité du mélange de (B), (C) et (D) s'élève à 0,001-0,09 Pa.s.

10. Procédé suivant les revendications 6 à 9, caractérisé en ce que la solution (II) est une solution alcaline d'un copolymère acide méthacrylique/ ester méthylique d'acide méthacrylique.